# EUROPÄISCHE PATENTSCHRIFT

(11) **EP 1 447 466 B1**
(45) Veröffentlichungstag und Bekanntmachung des Hinweises auf die Patenterteilung: **14.09.2005**
(21) Anmeldenummer: 03002932.6
(22) Anmeldetag: 10.02.2003
(51) Int. Cl.: D04H 3/08

(54) **Verfahren zur Herstellung eines Spinnvlieses aus Filamenten**
Process for making a spunbond nonwoven fabric from filaments
Procédé de fabrication d' un textile non-tissé filé-lié à partir de filaments

(43) Veröffentlichungstag der Anmeldung: 18.08.2004
(73) Patentinhaber: Reifenhäuser GmbH & Co. Maschinenfabrik, 53839 Troisdorf (DE)
(72) Erfinder: Sommer, Sebastian, 53844 Troisdorf (DE); Güdden, Jens, 53842 Spich (DE)
(74) Vertreter: Rohmann, Michael

(56) Entgegenhaltungen:
- EP-A- 0 473 325
- WO-A-96/02701
- US-A- 4 434 204

## Beschreibung

Die Erfindung betrifft ein Verfahren zur Herstellung eines Spinnvlieses aus Filamenten, insbesondere aus Filamenten aus thermoplastischem Kunststoff, wobei das Spinnvlies hydrodynamisch verfestigt wird. - Filamente meint im Rahmen der Erfindung Endlosfasern, d. h. theoretisch unendlich lange Fäden, aus denen das Spinnvlies gebildet wird. Davon zu unterscheiden sind sogenannte Stapelfasern, bei denen es sich um relativ kurze Fäden handelt. - Es liegt im Rahmen der Erfindung, dass aus den Filamenten kontinuierlich eine Spinnvliesbahn gebildet wird.

Aus der Praxis sind hydrodynamisch verfestigte Spinnvliese aus Filamenten bzw. Endlosfasern bekannt, die im Vergleich zu entsprechenden Stapelfaservliesen aus Kurzfasern eine schlechtere Einbindung der Fasern in das Vlies bzw. eine deutlich geringere Verschlaufung der Fasern bzw. Filamente aufweisen. Aus diesem Grunde zeichnen sich diese Spinnvliese durch nachteilhafte Vlieseigenschaften wie relativ geringe Festigkeiten, insbesondere eine relativ geringe Zugfestigkeit aus. Außerdem zeigen diese Spinnvliese eine unerwünschte hohe Abrasionsneigung. Diese Nachteile versucht man durch eine entsprechend intensive hydrodynamische Verfestigung zu vermeiden. Dadurch wird aber ein nachteilhaft hoher Aufwand, insbesondere Energieaufwand erforderlich und die resultierende Produktionsgeschwindigkeit lässt zu wünschen übrig.

Demgegenüber liegt der Erfindung das technische Problem zugrunde, ein Verfahren der eingangs genannten Art anzugeben, mit dem hydrodynamisch verfestigte Spinnvliese mit geringem Aufwand und mit hoher Produktionsgeschwindigkeit hergestellt werden können, die sich durch eine effektive Einbindung bzw. Verschlaufung der Fasern auszeichnen und hervorragende Vlieseigenschaften aufweisen.

Zur Lösung dieses technischen Problems lehrt die Erfindung ein Verfahren zur Herstellung eines Spinnvlieses aus Filamenten,
wobei die Filamente mit zumindest einem Netzmittel behandelt werden,
und wobei das aus den Filamenten gebildete Spinnvlies anschließend hydrodynamisch verfestigt wird.

Es liegt im Rahmen der Erfindung, dass aus den Filamenten zunächst ein Spinnvlies gebildet wird und das Spinnvlies dann mit dem Netzmittel behandelt wird. Behandlung mit einem Netzmittel meint im Rahmen der Erfindung, dass die Filamente bzw. das Spinnvlies in Kontakt mit dem Netzmittel gebracht wird.

Nach sehr bevorzugter Ausführungsform der Erfindung wird zumindest ein Tensid als Netzmittel eingesetzt. Ein solches erfindungsgemäß verwendetes Tensid weist einen lipophilen und einen hydrophilen Anteil bzw. ein lipophiles und ein hydrophiles Ende auf. Tenside haben sich im Rahmen des erfindungsgemäßen Verfahrens zur Behandlung der Filamente bzw. zur Behandlung des Spinnvlieses besonders bewährt. Als Netzmittel können ionische, d. h. kationische und/oder anionische Tenside eingesetzt werden oder auch nicht-ionische Tenside. Nach einer Ausführungsform der Erfindung werden Ampho-Tenside als Netzmittel verwendet.

Im Anschluss an die Behandlung mit dem Netzmittel, vorzugsweise mit dem zumindest einen Tensid wird das Spinnvlies hydrodynamisch verfestigt. Es liegt dabei im Rahmen der Erfindung, dass das Spinnvlies durch Wasserstrahlbehandlung verfestigt wird. Bei einer solchen Wasserstrahlverfestigung bzw. Wasserstrahlvernadelung verfestigen feine, sehr schnelle Wasserstrahlen den Vliesstoff.

Die Filamente werden zweckmäßigerweise mit einer geeigneten Spinerette erzeugt, der auf übliche Weise vorbereiteter thermoplastifizierter Kunststoff zugeführt wird. Aus dieser Spinerette treten dann die entsprechenden Filamente bzw. Endlosfäden aus. Es liegt im Rahmen der Erfindung, dass diese Filamente anschließend gekühlt werden. Es liegt fernerhin im Rahmen der Erfindung, dass die Filamente verstreckt werden. Zweckmäßigerweise werden die Filamente auf einer als endlos umlaufendes Ablegesiebband ausgebildeten Ablage zum Spinnvlies bzw. zur Spinnvliesbahn abgelegt. Vorzugsweise ist unter dem Ablegesiebband zumindest eine Saugeinrichtung bzw. ein Sauggebläse zum Ansaugen von Luft vorgesehen. Auf diese Weise wird in an sich bekannter Weise eine funktionssichere Ablage des Spinnvlieses erreicht. Das auf diese Weise gebildete Spinnvlies wird dann mit dem zumindest einen Netzmittel, bevorzugt mit zumindest einem Tensid behandelt und im Anschluss daran hydrodynamisch verfestigt.

Nach einer Ausführungsform kann das Spinnvlies noch auf der Ablage bzw. auf dem Ablegesiebband mit dem zumindest einen Netzmittel behandelt werden. Es liegt im Rahmen der Erfindung, dass das Spinnvlies zunächst in einem Kalander vorverfestigt wird und anschließend mit zumindest einem Netzmittel behandelt wird. Zweckmäßigerweise erfolgt nach der Behandlung mit dem Netzmittel zunächst eine Trocknung des Spinnvlieses. Nach einer Ausführungsform wird nach der Trocknung zunächst eine gewisse Reifezeit abgewartet. Im Anschluss daran erfolgt die erfindungsgemäße Wasserstrahlvernadelung des Spinnvlieses.

Gegenstand der Erfindung ist auch ein Verfahren zur Herstellung eines Mehrschichtproduktes (Laminates), wobei zumindest ein mit zumindest einem Netzmittel behandeltes Spinnvlies mit zumindest einer weiteren Schicht verbunden wird und wobei das Aggregat aus dem zumindest einen Spinnvlies und der zumindest einen weiteren Schicht anschließend hydrodynamisch verfestigt wird. Vorzugsweise wird als weitere Faserschicht eine Schicht aus hydrophilen, wasseraufnahmefähigen Fasern, insbesondere Zellulosefasern eingesetzt. Nach einer sehr bevorzugten Ausführungsform wird auf ein mit zumindest einem Netzmittel behandeltes Spinnvlies eine Schicht aus hydrophilen Fasern, vorzugsweise Pulpfasern aufgebracht und auf diese Schicht aus den hydrophilen Fasern wird ein weiteres mit zumindest einem Netzmittel behandeltes Spinnvlies aufgebracht. Anschließend wird das Aggregat aus den beiden Spinnvliesen und der Schicht aus hydrophilen Fasern hydrodynamisch verfestigt. Dabei liegt es auch im Rahmen der Erfindung, dass zunächst das Aggregat aus dem ersten Spinnvlies und den hydrophilen Fasern hydrodynamisch verfestigt wird und dass dann das zweite Spinnvlies auf die Schicht aus hydrophilen Fasern aufgebracht wird und im Anschluss daran das gesamte Aggregat noch einmal hydrodynamisch verfestigt wird.

Der Erfindung liegt die Erkenntnis zugrunde, dass mit der Merkmalskombination des erfindungsgemäßen Verfahrens (Behandlung mit dem Netzmittel und nachfolgende hydrodynamische Verfestigung bzw. Wasserstrahlbehandlung) Spinnvliese mit überraschend vorteilhaften Eigenschaften auf einfache und wenig aufwendige Weise hergestellt werden können. Durch einfache und zügig durchzuführende Maßnahmen wird eine hervorragende Einbindung der Filamente in den Vliesstoff bzw. eine optimale Verschlaufung der Filamente erzielt. Die erfindungsgemäß hergestellten Spinnvliese zeichnen sich gegenüber den aus dem Stand der Technik mit vergleichbarem Aufwand hergestellten hydrodynamisch verfestigten Spinnvliesen durch wesentlich verbesserte Festigkeiten, insbesondere durch eine höhere Zugfestigkeit aus. Im Übrigen ist die Abrasionsempfindlichkeit der erfindungsgemäß hergestellten Spinnvliese überraschenderweise deutlich geringer als bei den vorgenannten bekannten Spinnvliesen. Im Ergebnis können mit dem erfindungsgemäßen Verfahren die mechanischen Eigenschaften von Spinnvliesen beachtlich verbessert werden. - Andererseits können mit dem erfindungsgemäßen Verfahren hergestellte Spinnvliese, die im Vergleich zu nach dem Stand der Technik hergestellten hydrodynamisch verfestigten Spinnvliesen vergleichbare Eigenschaften aufweisen, mit deutlich geringerem Energieaufwand bei der Verfestigung sowie mit höherer Produktionsgeschwindigkeit hergestellt werden. Diese beachtlichen Vorteile konnte der Fachmann im Lichte des Standes der Technik keinesfalls erwarten.

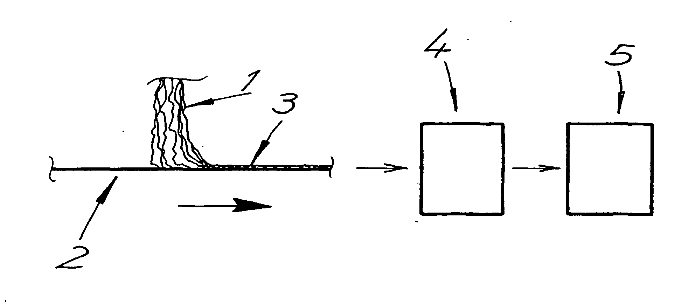

Nachfolgend wird die Erfindung anhand einer lediglich ein Ausführungsbeispiel darstellenden Zeichnung näher erläutert. Die einzige Figur zeigt ganz schematisch eine Vorrichtung zur Durchführung des erfindungsgemäßen Verfahrens. Die Filamente 1 werden auf einer als endlos umlaufendes Ablegesiebband 2 ausgebildeten Ablage zur Spinnvliesbahn 3 abgelegt. Im Ablegebereich der Filamente 1 ist unterhalb des Ablagesiebbandes 2 zweckmäßigerweise eine nicht dargestellte Saugeinrichtung vorgesehen, mit welcher Saugeinrichtung Luft durch das Ablegesiebband 2 angesaugt wird.

Das auf die vorstehend beschriebene Weise hergestellte Spinnvlies wird dann in einer Behandlungsstation 4 mit einem Netzmittel, vorzugsweise mit einem Tensid behandelt. Im Anschluss daran erfolgt in einer Verfestigungsvorrichtung 5 eine Vliesverfestigung durch Wasserstrahlbehandlung bzw. durch Behandlung mit Hochdruckwasserstrahlen. Die auf diese Weise gebildete Spinnvliesbahn 3 zeichnet sich durch hervorragende Eigenschaften, insbesondere durch hohe Festigkeiten und geringe Abrasionsneigung aus.

## Patentansprüche

1. Verfahren zur Herstellung eines Spinnvlieses aus Filamenten,
wobei die Filamente mit zumindest einem Netzmittel behandelt werden,
und wobei das aus den Filamenten gebildete Spinnvlies anschließend hydrodynamisch verfestigt wird.

2. Verfahren nach Anspruch 1, wobei die Filamente mit einer Spinerette erzeugt und anschließend gekühlt werden.

3. Verfahren nach einem der Ansprüche 1 oder 2, wobei die Filamente auf einem kontinuierlich bewegten Ablegesiebband abgelegt werden.

4. Verfahren nach einem der Ansprüche 1 bis 3, wobei im Ablegebereich der Filamente unterhalb des Ablegesiebbandes zumindest eine Saugeinrichtung vorgesehen ist, mit welcher Saugeinrichtung Luft durch das Ablegesiebband angesaugt wird.

5. Verfahren nach einem der Ansprüche 1 bis 4, wobei die Filamente zum Spinnvlies abgelegt werden und das Spinnvlies im Anschluss daran mit dem Netzmittel behandelt wird.

6. Verfahren nach einem der Ansprüche 1 bis 5, wobei zumindest ein Tensid als Netzmittel eingesetzt wird.

7. Verfahren nach einem der Ansprüche 1 bis 6, wobei das Spinnvlies durch Wasserstrahlbehandlung hydrodynamisch verfestigt wird.

## Claims

1. Method of producing a spunbonded nonwoven web from filaments,
wherein the filaments are treated with at least one wetting agent,
and wherein the spunbonded nonwoven web formed from the filaments is then hydrodynamically consolidated.

2. Method according to Claim 1, wherein the filaments are produced by a spinneret and are then cooled.

3. Method according to either of Claims 1 and 2, wherein the filaments are collected on a continuously moving sieve-like collecting belt.

4. Method according to one of Claims 1 to 3, wherein at least one suction device is provided below the sieve-like collecting belt in the region where the filaments are collected on said belt, by means of which suction device air is drawn through the sieve-like collecting belt.

5. Method according to one of Claims 1 to 4, wherein the filaments are collected to form the spunbonded nonwoven web and the spunbonded nonwoven web is then treated with the wetting agent.

6. Method according to one of Claims 1 to 5, wherein at least one surfactant is used as the wetting agent.

7. Method according to one of Claims 1 to 6, wherein the spunbonded nonwoven web is hydrodynamically consolidated by means of a water jet treatment.

## Revendications

1. Procédé de fabrication d'un filé-lié formé de filaments,
dans lequel les filaments sont traités par au moins un agent mouillant,
et dans lequel le filé-lié formé de filaments est ensuite consolidé par voie hydrodynamique.

2. Procédé suivant la revendication 1, dans lequel les filaments sont produits par une fileuse et sont ensuite refroidis.

3. Procédé suivant l'une des revendications 1 et 2, dans lequel les filaments sont déposés sur une toile perforée déplacée en continu.

4. Procédé suivant l'une des revendications 1 à 3, dans lequel au moins un dispositif d'aspiration, par lequel de l'air est aspiré au travers de la toile perforée, est prévu dans la zone de dépôt des filaments au-dessous de la toile perforée.

5. Procédé suivant l'une des revendications 1 à 4, dans lequel les filaments sont déposés en un filé-lié, le filé-lié étant ensuite traité par l'agent mouillant.

6. Procédé suivant l'une des revendications 1 à 5, dans lequel au moins un agent tensio-actif est utilisé comme agent mouillant.

7. Procédé suivant l'une des revendications 1 à 6, dans lequel le filé-lié est consolidé hydrodynamiquement par traitement au jet d'eau.
